# EUROPÄISCHE PATENTSCHRIFT

(11) **EP 1 476 482 B1**
(45) Veröffentlichungstag und Bekanntmachung des Hinweises auf die Patenterteilung: **19.03.2008**
(21) Anmeldenummer: 03714724.6
(22) Anmeldetag: 04.02.2003
(51) Int. Cl.: C08F 283/06

(54) **PFROPFCOLPOLYMERE IN KOSMETISCHEN FORMULIERUNGEN**
GRAFT COPOLYMERS IN COSMETIC FORMULATIONS
COPOLYMERES GREFFES POUR PREPARATIONS COSMETIQUES

(30) Priorität: 15.02.2002 DE 10206596
(43) Veröffentlichungstag der Anmeldung: 17.11.2004
(73) Patentinhaber: BASF SE, 67056 Ludwigshafen (DE)
(72) Erfinder: MATHAUER, Klemens, 69115 Heidelberg (DE); JAHNEL, Wolfgang, 76756 Bellheim (DE); HÖSSEL, Peter, 67105 Schifferstadt (DE)
(86) Internationale Anmeldenummer: PCT/EP2003/001064
(87) Internationale Veröffentlichungsnummer: WO 2003/068834

(56) Entgegenhaltungen:
- EP-A- 1 138 322
- WO-A-02/15854
- WO-A-96/34903

## Beschreibung

Die Erfindung betrifft die Verwendung von Pfropfcopolymerisaten als Bestandteil in kosmetischen Mitteln. Die Pfropfcopolymerisate entstehen dabei durch Pfropfung von monoethylenisch ungesättigten, offenkettigen N-Vinylamideinheiten enthaltenden Monomeren auf eine polymere Pfropfgrundlage.

Polymere finden in der Haarkosmetik vielfache Anwendung. Ihre Aufgabe in der Haarkosmetik besteht darin, die Eigenschaften des Haares zu beeinflussen, insbesondere dem Haar Festigung zu geben, die Kämmbarkeit zu verbessern und ein angenehmes Griffgefühl zu vermitteln.

So werden Conditioner dazu eingesetzt, um die Trocken- und Nasskämmbarkeit, Gefühl, Glanz und Erscheinungsform zu verbessern sowie dem Haar antistatische Eigenschaften zu verleihen. Bevorzugt werden wasserlösliche Polymere mit polaren, häufig kationischen Funktionalitäten eingesetzt, die eine größere Affinität zur strukturell bedingten negativen Oberfläche des Haares aufweisen. Struktur und Wirkungsweise verschiedener Haarbehandlungspolymere sind in Cosmetic & Toiletries 103 (1988) 23 beschrieben. Handelsübliche Conditionerpolymere sind z.B. kationische Hydroxyethylcellulose, kationische Polymere auf der Basis von N-Vinylpyrrolidon, z.B. Copolymere aus N-Vinylpyrrolidon und quarterniertem N-Vinylimidazol, Acrylamid und Diallyldimethylammoniumchlorid oder Silikone.

Zur Festigung von Haarfrisuren werden Vinyllactam-Homo- und Copolymere und Carboxylatgruppenhaltige Polymere eingesetzt. Anforderungen an Haarfestigerharze sind zum Beispiel eine starke Festigung bei hoher Luftfeuchtigkeit, Elastizität, Auswaschbarkeit vom Haar, Verträglichkeit in der Formulierung und ein angenehmer Griff des Haares.

Schwierigkeiten bereitet oft die Kombination verschiedener Eigenschaften wie zum Beispiel starke Festigung, angenehmer Griff des Haares und gleichzeitige verdickende Wirkung der Polymere in den haarkosmetischen Zubereitungen.

Dies ist insbesondere in Gelformulierungen von Bedeutung. Darüberhinaus zeigen übliche Festigerpolymere häufig Unverträglichkeiten mit Verdickerpolymeren, es kommt zu Trübungen und Ausfällungen in den kosmetischen Formulierungen. Klassische Verdicker, die entweder aus vernetzter Polyacrylsäure (Carbopol) oder Copolymeren bestehen, haben den Nachteil, daß sie aufgrund der Vernetzung keine für die Haarfestigung geeigneten Filme bilden. Sie sorgen für die Konsistenz des Gels, werden aber nach dem Trocknen des Gels auf dem Haar nicht mehr benötigt und stören daher potentiell die anwendungstechnischen Eigenschaften der Formulierung (Festigungswirkung, Feuchtigkeitsempfindlichkeit).

Die WO-A-96/03969 beschreibt Haarpflegemittel, enthaltend ein N-Vinylformamid-Homopolymer oder ein Copolymer aus N-Vinylformamideinheiten und einem weiteren Vinylmonomer, ausgewählt unter Styrolen, Alkylestern von Acryl- und Methacrylsäure, Vinylestern der Formel CH₂=CH-OCO-Alkyl, N-Alkyl-substituierten Acryl- und Methacrylamiden, Estern von Fumar-, Ithacon- und Maleinsäure, Vinylethern, Hydroxy-funktionalisierten Acrylaten und Methacrylaten, Acrylamid, nicht-Alkyl-substituierten Acrylamiden und cyclischen Amiden. Als konkretes Beispiel für ein cyclisches Amid wird N-Vinylpyrrolidon genannt. Weitere Beispiele für Vinylmonomere sind sekundäre, tertiäre und quarternäre Amine, wie Dimethyl-diallylammoniumchlorid, Dimethylaminoethylmethacrlyat oder Dimethylaminopropyl-methacrylat.

DE 19640363 beschreibt Copolymere aus N-Vinylformamid und quarterniertem N-Vinylimidazol sowie deren Anwendungen in der Kosmetik.

DE 19907587.5 beschreibt die Verwendung von Polymerisaten, die erhältlich sind durch radikalische Polymerisation von mindestens einem Vinylester in Gegenwart von polyetherhaltigen Verbindungen und gegebenenfalls eines oder mehreren copolymerisierbaren Monomeren und anschließender zumindest teilweiser Verseifung der Esterfunktion in haarkosmetischen Formulierungen. Als copolymerisierbare Monomere ist u.a. Vinylformamid genannt.

Die DE-A1-44 09 903 beschreibt N-Vinyleinheiten enthaltende Pfropfpolymerisate, Verfahren zu ihrer Herstellung und ihre Verwendung. Dabei werden auf einer Pfropfgrundlage, welches ein Polymerisat ist, das jeweils mindestens 5 Gew.-% Einheiten der Formeln 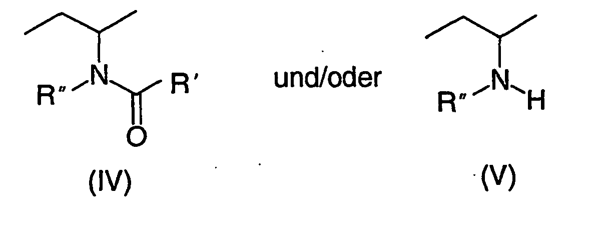 enthält, wobei R', R" = H oder C₁- bis C₆-Alkyl bedeuten, monoethylenisch ungesättigte Monomere aufgepfropft. Als monoethylenisch ungesättigte Monomere kommen alle ethylenisch ungesättigten Monomere in Frage, deren Polymerisation durch die Amingruppen in freier oder in Salzform nicht inhibiert wird, wie' z.B. monoethylenisch ungesättigte Mono- und Dicarbonsäuren, deren Salze und Ester mit C₁- bis C₃₀-Alkoholen. Eine Eignung dieser Pfropfcopolymere als Wirkstoff in kosmetischen Formulierungen wird nicht genannt.

Die WO 96/34903 beschreibt N-Vinyleinheiten enthaltende Pfropfpolymerisate, Verfahren zu ihrer Herstellung und ihre Verwendung. Dabei werden auf einer Pfropfgrundlage, welches ein Polymerisat ist, das mindestens 3 Einheiten eines C₂- bis C₄-Alkylenoxids enthalten, und/oder Polytetrahydrofuran enthalten, monoethylenisch ungesättigte Monomere aufgepfropft und anschließend zumindest teilweise verseift. Eine Eignung dieser Pfropfcopolymere als Wirkstoff in kosmetischen Formulierungen wird nicht genannt. Pfropfpolymerisate gemäß Anspruch 1 werden nicht beschrieben.

Aus der US-A-5 334 287 sind Pfropfpolymerisate bekannt, die durch radikalisch initiierte Polymerisation von N-Vinylcarbonsäureamiden, vorzugsweise N-Vinylformamid, und gegebenenfalls anderen Monomeren in Gegenwart von Monosacchariden, Oligosacchariden, Polysacchariden oder jeweils deren Derivaten und gegebenenfalls Hydrolyse der einpolymerisierten Gruppe N-Vinylcarbonsäureamide unter Bildung von Vinylamineinheiten erhältlich sind. Eine Eignung dieser Pfropfcopolymere als Wirkstoff in kosmetischen Formulierungen wird nicht genannt.

In der WO 9825981 werden amphiphile Pfropfpolymere durch Pfropfen hydrophober Monomerer, wie z.B. Styrol, auf Polymere die Strukturelemente der Formel (IV) und/oder (V) enthalten synthetisiert. Die dabei erhaltenen Pfropfpolymeren werden u.a. als Zusatzstoffe zu kosmetischen Formulierungen verwendet.

In der DE-A1-196 40 363 wird die Verwendung wasserlöslicher Copolymere als Wirkstoff in kosmetischen Formulierungen beansprucht. Als charakteristisches Strukturelement enthält das Copolymer Einheiten der allgemeinen Formel (VI) und 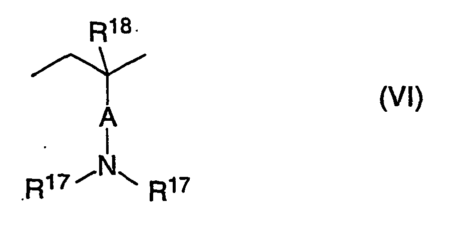 worin A für eine chemische Bindung oder eine Alkylengruppe steht, die Reste R¹⁷ unabhängig voneinander für H, Alkyl, Cycloalkyl, Aryl oder Aralkyl und R¹⁸ für H, Alkyl oder Aralkyl stehen.

Körperpflegecremes, die ein Monoaldehyd-modifiziertes Vinylaminpolymer enthalten, sind aus der US 5 270 379 bekannt.

Copolymere, die beispielsweise als Haarfestiger Verwendung finden und aufgebaut sind aus N-Vinylamidmonomeren der Formel 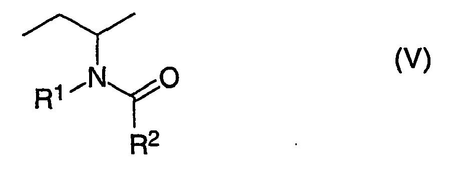 worin R1 und R2 für H oder C₁-C₅-Alkyl stehen und das Comonomer ausgewählt ist unter Vinylethern, Vinyllactamen, Vinylhalogeniden, Vinylestern einbasiger gesättigter Carbonsäuren, (Meth)acrylsäureestern, -amiden und -nitrilen und estern, Anhydriden und Imiden der Maleinsäure sind aus der DE 14 95 692 bekannt.

In der US 4 713 236 sind Haarconditioner auf Basis von Vinylamineinheiten enthaltenden Polymeren beschrieben. Genannt sind dabei insbesondere Polyvinylamin sowie dessen Salze, α-substituierte Polyvinylamine wie z.B. Poly-(α-aminoacrylsäure) oder auch Copolymere, die neben Vinylamin Comonomere wie Vinylalkohol, Acrylsäure, Acrylamid, Maleinsäureanhydrid, Vinylsulfonat und 2-Acrylamido-2-methylpropan-sulfonsäure einpolymerisiert enthalten.

Aufgabe der vorliegenden Erfindung war es Polymere zu finden, die für kosmetische Anwendungen gut geeignet sind und beispielsweise im Gebiet Haarkosmetik gute anwendungstechnische Eigenschaften wie angenehmen Griff und gleichzeitig gute Konditionierwirkung bzw. eine gute Festigungswirkung und dabei gleichzeitig eine verdickende Eigenschaft aufweisen. Hierzu war insbesondere die Klarlöslichkeit der Polymere in üblichen haarkosmetischen Zubereitungen von Interesse. Aufgabe war die Bereitstellung von Polymeren, die sowohl haarkosmetischen Anforderungen (Festigungswirkung, Curlretention) als auch anwendungstechnischen Anforderungen (verdickende Eigenschaften, Klarlöslichkeit) genügen.

Aufgabe der vorliegenden Erfindung bestand darin Copolymere zu finden, die eine Verdickungswirkung (nach Neutralisation des Polymers mit für die Kosmetik üblichen Basen) mit filmbildenden Eigenschaften verbinden, so daß diese Polymere z.B. in Haargelformulierungen als Festigerpolymer verwendet werden kann.

Besonderes Interesse bestand an Polymeren, welche verdickende Eigenschaften auch ohne den Zusatz weiterer, üblicher Verdicker (z.B. hochmolekularer Polyacrylsäuren vom Typ Carbopole^{®}) aufweisen.

Die Aufgabe wurde erfindungsgemäß gelöst durch Pfropfcopolymerisate, die erhältlich sind durch radikalische Pfropfcopolymerisation von
a) N-Vinylformamid,
b) eines oder mehreren copolymerisierbaren Monomeren der allgemeinen Formel (I) 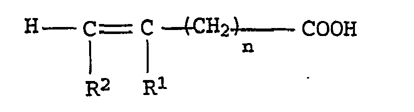
wobei n= 0,1 und R¹ und R² unabhängig voneinander = H, C₁-C₄ Alkyl, CN, COOH darstellen,
und/oder einer oder mehreren ungesättigten Sulfonsäuren
und/oder einer oder mehreren ungesättigten Phosphonsäuren
auf eine polymere Pfropfgrundlage c) der allgemeinen Formel

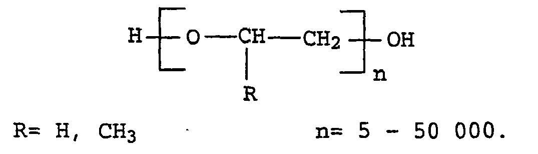

Bei der Herstellung der erfindungsgemäß verwendeten Polymerisate kann es während der Polymerisation zu einer Pfropfung auf die polymere Pfropfgrundlage c) kommen, was zu den vorteilhaften Eigenschaften der Polymerisate führen kann. Es sind jedoch auch andere Mechanismen als Pfropfung vorstellbar.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften Verbindungen c) und Homo- oder Copolymerisaten der Monomeren a) und b) zu verstehen.

Die erfindungsgemäßen Polymerisate sind vorzugsweise wasserlöslich bzw. wasserdispergierbar.

Unter wasserlöslichen Polymeren sollen hier Polymere verstanden werden, die sich zu mindestens 1 g/l bei 20°C in Wasser lösen. Unter wasserdispergierbaren Polymeren sollen hier Polymere verstanden werden, die unter Rühren in dispergierbare Partikel zerfallen.

Zur Herstellung der erfindungsgemäßen Polymerisate wird als Monomer a) N-Vinylformamid eingesetzt.

Als Monomer b) werden eines oder mehrere copolymerisierbare Monomere eingesetzt,
der allgemeinen Formel (I) 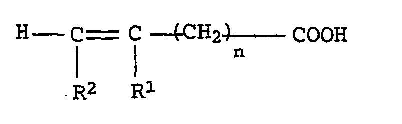 wobei n= 0,1 und R¹ und R² unabhängig voneinander = H, C₁-C₄ Alkyl, CN, COOH darstellen,
und/oder eine oder mehrere ungesättigte Sulfonsäuren und/oder eine oder mehrere ungesättigte Phosphonsäuren.

Besonders bevorzugt sind Monomere der allgemeinen Formel (I).

Geeignete C₁-C₄ Alkylreste sind z.B. Methyl, Ethyl, n-Propyl, Isopropyl, n-Butyl, t-Butyl.

Als Beispiele für Monomere der allgemeinen Formel (I) seien genannt Acrylsäure, Methacrylsäure, Maleinsäure, Fumarsäure, Crotonsäure, Isocrotonsäure, Vinylessigsäure und Itaconsäure.

Besonders bevorzugt sind Monomere der allgemeinen Formel (I) mit n=0. Insbesondere bevorzugt sind Acrylsäure und Methacrylsäure.

Als ungesättigte Sulfonsäuren kommen alle Verbindungen der Formel R-SO₃H in Betracht., wobei R für einen Rest mit mindestens einer ethylenisch ungesättigten Gruppe steht.

Besonders geeignet als ungesättigte Sulfonsäuren sind Verbindungen der allgemeinen Formel (II) 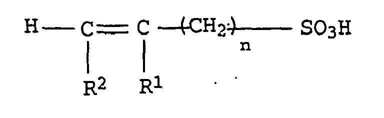 wobei n= 0, 1 und R¹ und R² unabhängig voneinander = H, C₁-C₄ Alkyl, CN, COOH darstellen.

Beispiele für Monomere der allgemeinen Formel (II) sind Vinylsulfonsäure und Methallylsulfonsäure.

Geeignet sind weiterhin aromatische ungesättigte Sulfonsäuren wie beispielsweise m-Styrolsulfonsäure und/oder p-Styrolsulfonsäure.

Exemplarisch seien für weitere geeignete ungesättigte Sulfonsäuren genannt Arylamidopropansulfonsäure

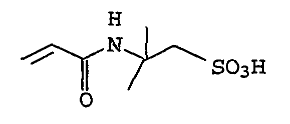

### Sulfoethylmethacrylat

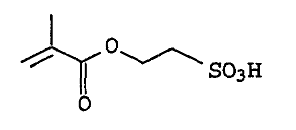

sowie

### Acrylsäure(3-sulfopropyl)ester

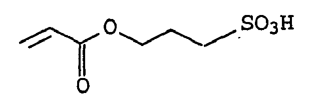

und Acrylsäure(2-sulfopropyl)ester.

Als ungesättigte Phosphonsäuren kommen alle Verbindungen der Formel R-PO₃H in Betracht, wobei R für einen Rest mit mindestens einer ethylenisch ungesättigten Gruppe steht.

Besonders geeignet als ungesättigte Phosphonsäuren sind Verbindungen der allgemeinen Formel (III) 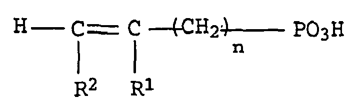 wobei n= 0, 1 und R¹ und R² unabhängig voneinander = H, C₁-C₄ Alkyl, CN, COOH darstellen.

Beispiel für ein Monomer der allgemeinen Formel (III) ist Vinylphosphonsäure.

Die genannten Monomere können einzeln oder in Mischung eingesetzt werden.

Die polymere Pfropfgrundlage c) wird ausgewählt aus Verbindungen der allgemeinen Formel

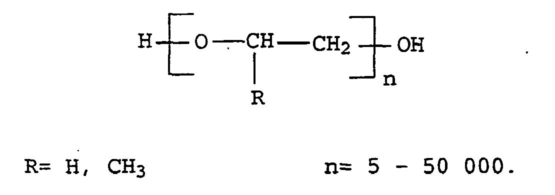

Das Molekulargewicht der Polyether liegt im Bereich zwischen 200 und 2 500 000 (nach Zahlenmittel), bevorzugt im Bereich von 300 bis 1 000 000, besonders von 400 bis 100 000, besonders bevorzugt im Bereich von 500 bis 50 000, ganz besonders bevorzugt im Bereich von 800 bis 40 000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommt Propylenoxid in Betracht. Der Ethylenoxidanteil der Copolymerisate beträgt vorzugsweise 40 bis 99 mol-%, der Propylenoxidanteil 1 bis 60 mol-%.

Die Herstellung der Pfropfgrundlage c) erfolgt nach bekannten Verfahren, zum Beispiel der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200, vorzugsweise 40 bis 110°C. Geeignete Initiatoren sind beispielsweise Azö- und Peroxyverbindungen sowie die üblichen Redoxinitiatorsysteme, wie Kombinationen aus Wasserstoffperoxid und reduzierend wirkenden Verbindungen, zum Beispiel Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden. Bevorzugt werden silikonfreie Regler eingesetzt.

In einer bevorzugten Ausführungsform werden die Polymerisate in Gegenwart eines Vernetzers d) hergestellt.

Als vernetzende Monomere d) können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer d) sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octa-decen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer d) sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Ferner geeignet sind Amide von ungesättigten Carbonsäuren, wie z.B., Acryl- und Methacrylsäure, Itaconsäure, Maleinsäure, und N-Allylaminen von mindestens zweiwertigen Aminen, wie zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z.B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Ganz besonders bevorzugt als Vernetzer sind Pentaerythrittriallylether, Allylether von Zuckern wie Saccharose, Glucose, Mannose, Divinylbenzol, Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin.

Der Anteil der vernetzend wirkenden Monomeren beträgt 0 bis 10 Gew.-%, bevorzugt 0 bis 5 Gew.-%, ganz besonders bevorzugt 0 bis 2 Gew.-%.

Bei der Polymerisation zur Herstellung der erfindungsgemäßen Polymerisate können gegebenenfalls auch andere Polymere, wie zum Beispiel Polyamide, Polyurethane, Polyester, Homo- und Copolymere von ethylenisch ungesättigten Monomeren, zugegen sein. Beispiele für solche zum Teil auch in der Kosmetik eingesetzten Polymeren sind die unter den Handelsnamen bekannten Polymere Amerhold^{™}, Ultrahold^{™}, Ultrahold Strong^{™}, Luviflex^{™} VBM, Luvimer^{™}, Acronal^{™}, Acudyne^{™}, Stepanhold^{™}, Lovocryl^{™}, Versatyl^{™}, Amphomer^{™} oder Eastma AQ^{™}.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Zur Herstellung der Polymerisate können die Monomeren der Komponente a) und der Komponente b) in Gegenwart der Pfopfgrundlage c) sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidonopropan)dihydrochlorid oder 2-2'-Azo-bis-(2-methylbutyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat.

Bevorzugt werden organische Peroxide eingesetzt.

Die Polymerisation kann auch durch Einwirkung von ultravioletter Strahlung, gegebenenfalls in Gegenwart von UV-Initiatoren, durchgeführt werden. Für das Polymerisieren unter Einwirkung von UV-Strahlen setzt man die dafür üblicherweise in Betracht kommenden Photoinitiatoren bzw. Sensibilisatoren ein. Hierbei handelt es sich beispielsweise um Verbindungen wie Benzoin und Benzoinether, α-Methylbenzoin oder α-Phenylbenzoin. Auch sogenannte Triplett-Sensibilisatoren, wie Benzyldiketale, können verwendet werden. Als UV-Strahlungsquellen dienen beispielsweise neben energiereichen UV-Lampen, wie Kohlenbogenlampen, Quecksilberdampflampen oder Xenonlampen auch UV-arme Lichtquellen, wie Leuchtstoffröhren mit hohem Blauanteil.

Die verwendeten Mengen an Initiator bzw. Initiatorgemischen bezogen auf eingesetztes Monomer liegen zwischen 0,01 und 10 Gew.-%, vorzugsweise zwischen 0,1 und 5 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar, ablaufen.

Die Polymerisation kann beispielsweise als Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation durchgeführt werden, ohne daß die verwendbaren Methoden darauf beschränkt sind. Besonders bevorzugt wird die Polymerisation als Fällungspolymerisation durchgeführt.

Bei der Polymerisation in Substanz kann man so vorgehen, daß man die Pfopfgrundlage c) in dem Monomer der Gruppe a) (N-Vinylformamid) und weiteren Comonomeren der Gruppe b) löst und nach Zugabe eines Polymerisationsinitiators die Mischung auspolymerisiert. Die Polymerisation kann auch halbkontinuierlich durchgeführt werden, indem man zunächst einen Teil, z.B. 10 % des zu polymerisierenden Gemisches aus der Pfropfgrundlage c), dem Monomeren a), weiteren Comonomeren der Gruppe b) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die Pfopfgrundlag c) in einem Reaktor vorlegt, auf die Polymerisationstemperatur erwärmt und das Monomer der Gruppe a), weiteren Comonomeren der Gruppe b) und Polymerisationsinitiator entweder auf einmal, absatzweise oder vorzugsweise kontinuierlich zufügt und polymerisiert.

Falls gewünscht, kann die oben beschriebene Polymerisation auch in einem Lösemittel durchgeführt werden. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Polymerisation kann auch in Wasser als Lösemittel durchgeführt werden. In diesem Fall liegt zunächst eine Lösung vor, die in Abhängigkeit von der Menge der zugegebenen Monomeren der Komponente a) in Wasser mehr oder weniger gut löslich ist. Um wasserunlösliche Produkte, die während der Polymerisation entstehen können, in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Polymerisation in Wasser so verfahren, daß man die wasserunlöslichen Polymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im Bereich von 3 bis 13 liegt. Zur Definition des HLB-Werts wird auf die Veröffentlichung von W.C. Griffin, J. Soc. Cosmetic Chem., Band 5, 249 (1954), hingewiesen.

Die Menge an Tensiden, bezogen auf das Polymerisat, beträgt 0,1 bis 10 Gew.-%. Bei Verwendung von Wasser als Lösemittel erhält man Lösungen bzw. Dispersionen der Polymerisate. Sofern man Lösungen des Polymerisates in einem organischen Lösemittel herstellt bzw. in Mischungen aus einem organischen Lösemittel und Wasser, so verwendet man pro 100 Gew.-Teile des Polymerisates 5 bis 2000, vorzugsweise 10 bis 500 Gew.-Teile des organischen Lösemittels oder des Lösemittelgemisches.

Bevorzugt sind Polymere, die erhältlich sind durch radikalische Pfropfcopolymerisation von

| | |
|---|---|
| - 10 - 90 Gew.-% | N-Vinylformamid |
| - 1 - 60 Gew.-% | eines oder mehreren copolymerisierbaren Monomeren b), insbesondere der Monomere der allgemeinen Formel (I) |
| - 10 - 80 Gew.-% | polymere Pfropfgrundlage c), insbesondere mit einem Molekulargewicht von 500 bis 50 000 |
| - 0 - 10 Gew.-% | eines oder mehrerer vernetzend wirkender Monomere d) |

mit der Maßgabe, dass sich die Mengen zu 100 % addieren.

Bevorzugt sind Polymere, die erhältlich sind durch radikalische Pfropfcopolymerisation von

| | |
|---|---|
| - 20 - 80 Gew.-% | N-Vinylformamid |
| - 10 - 60 Gew.-% | eines oder mehreren copolymerisierbaren Monomeren b), insbesondere der Monomere der allgemeinen Formel (I) |
| - 20 - 70 Gew.-% | polymere Pfropfgrundlage c), insbesondere mit einem Molekulargewicht von 500 bis 50 000 |
| - 0 - 5 Gew.-% | eines oder mehrerer vernetzend wirkender Monomere d) |

mit der Maßgabe, dass sich die Mengen zu 100 % addieren.

Ganz besonders bevorzugt sind Polymere, die erhältlich sind durch radikalische Pfropfcopolymerisation von

| | |
|---|---|
| - 40 - 80 Gew.-% | N-Vinylformamid |
| - 10 - 40 Gew.-% | eines oder mehreren copolymerisierbaren Monomeren b), insbesondere der Monomere der allgemeinen Formel (I) |
| - 20 - 80 Gew.-% | polymere Pfropfgrundlage c), insbesondere mit einem Molekulargewicht von 500 bis 50 000 |
| - 0 - 2 Gew.-% | eines oder mehrerer vernetzend wirkender Monomere d) |

mit der Maßgabe, dass sich die Mengen zu 100 % addieren.

Die bevorzugte Methode zur Herstellung der erfindungsgemäßen Polymere ist die Fällungspolymerisation. Für diese Polymerisation werden Lösungsmittel verwendet, in denen die Ausgangsstoffe für die Polymerisation löslich und das entstehende Polymer unlöslich sind. Geeignete Lösungsmittel sind beispielsweise aromatische Kohlenwasserstoffe wie Toluol, Xylole, Benzol oder aliphatische Kohlenwasserstoffe wie n-Alkane, Cyclohexan, Ester der Essigsäure wie Ethylacetat, Butylacetat, Ether wie z.B. Diethylether, Dipropylether, Dibutylether, Methyl tert.-butylether, Diethylenglykoldimethylether, Ketone wie Aceton, Methylethylketon und Mischungen dieser Lösungsmittel. Mischungen von z.B. Ethylacetat und Butylacetat sind besonders geeignet, da in diesem Lösungsmittelgemisch die Polymere in leicht abtrennbare Form anfallen (Sedimentation ist beschleunigt) und außerdem die Reaktionstemperatur im Mischungen von Butylacetat und Ethylacetat oberhalb der Siedetemperatur von Ethylacetat gewählt werden kann bei gleichzeitiger Siedekühlung durch siedendes Ethylacetat.

Als Initiatoren können alle Initatoren Verwendung finden, wie sie auch bei der Lösungspolymeriation verwendet werden. Vorzugsweise werden 0,01 bis 1,5 Gew % Initiator bezogen auf die eingesetzen Monomere verwendet.

Die Fällungspolymerisation wird üblicherweise bei Temperaturen von 20 bis 150°C, bevorzugt 40 bis 120°C, insbesondere 60 bis 100°C durchgeführt.

Die Fällungspolymerisation wird üblicherweise bei Drücken von 1 bis 15 bar, insbesondere 1 bis 6 bar durchgeführt.

Das Lösungsmittel bzw. Lösungsmittelgemisch bestimmt durch die entsprechenden Siedetemperaturen die maximale Reaktionstemperatur sofern drucklos polymerisiert wird. Polymerisation unter Druck sind aber ebenfalls machbar.

Generell kann die Fällungspolymerisation bei Feststoffgehalten bis ca. 40% durchgeführt werden. Bevorzugt wird ein Bereich zwischen 25 und 40%. Besonders bei hohen Feststoffgehalten ist es ratsam, die Polymerisation in Gegenwart eines Schutzkolloid-Polymers durchzuführen. Geeignete Schutzkolloid-Polymere sind solche, die sich gut in den verwendeten Lösungsmitteln lösen und nicht mit den Monomeren reagieren. Geeignete Polymere sind z.b. Copolymere der Maleinsäure mit Vinylalkylethern und/oder Olefinen mit 8 bis 20 C-Atomen oder entsprechende Copolymere der Maleinsäurehalbester mit C10-C20 Alkoholen oder auch Mono- und Diamide der Maleinsäure mit C10 -C20 Alkylaminen sowie Polyvinylalkoholether mit Alkylgruppen, die 1 bis 20 C-Atome tragen oder auch Polyvinylmethyl, -ethyl, -isobutly oder -octadecylether. Die Menge an verwendetem Schutzkolloid-Polymer sind normalerweise zwischen 0,05 Gew% und 4 Gew% (bezogen auf Monomere), vorzugsweise zwischen 0,1 und 2 Gew%. Es ist oft vorteilhaft, Mischungen mehrerer Schutzkolloid-Polymere zu verwenden.

Die Polymerisation wird durchgeführt, in dem man Lösungsmittel, Pfropfgrundlage, Schutzkolloidpolymer und eventuell Vernetzer vorlegt, aufheizt, und die Polymerisation durch Zugabe von Inititator und Monomeren (eventuell gelöst im gleichen Lösungsmittel oder Lösungsmittelgemisch) durchführt. Man kann aber auch Teilmengen der Monomere und des Inititators vorlegen (z.B. 10%), dieses Gemisch auf Polymerisationstemperatur erhitzen und nach Anspringen der Reaktion den Rest der zu polymerisierenden Mischung nach Fortschritt der Polymeristion zugegen. Ebenso ist es möglich den verwendeten Vernetzer in Teilmengen oder gar nicht vorzulegen und den Rest zusammen mit den restlichen Komponenten. Bei niedrigeren Feststoffgehalten ist es auch denkbar, sämtliche Einsatzstoffe in einer Batchreaktion vorzulegen.

Monomer und Initiator werden im allgemeinen in einer Zeit zwischen 1 und 10 Stunden zudosiert, bevorzugt zwischen 2 und 5 Stunden.

Das ausgefallene Polymer wird anschließend aus der Reaktionsmischung isoliert, wofür jede allgemeine Methode zur Isolierung der Polymere in der konventionellen Fällungspolymerisation verwendet werden kann. Solche Methoden sind Filtration, Zentrifugation, Eindampfen des Lösungsmittels oder Kombinationen dieser Methoden. Zur weiteren Reinigung des Polymers von nichtpolymerisierten Bestandteilen wird das Polymer gewaschen. Dafür kann man prinzipiell die gleichen Lösungsmittel verwenden wie sie für die Polymerisation geeignet sind. Zur leichteren Trocknung der Polymere empfiehlt es sich aber, niedrig siedende Lösungsmittel wie z.B Aceton zu verwenden.

Die erfindungsgemäßen Pfropfcopolymerisate können im Anschluss an die Polymerisation verseift werden. Durch die Verseifung wird eine kationische Gruppe im Polymer erzeugt. Dies kann zu einer erhöhten Wasserlöslichkeit und verbesserten konditionierenden Eigenschaften in kosmetischen Anwendungen führen.

Aus den oben beschriebenen Pfropfcopolymerisaten erhält man durch teilweise oder vollständige Abspaltung der Formylgruppen, aus denen in das Polymerisat eingebautem N-Vinylformamid, unter Bildung von Amin- bzw. Ammoniumgruppen Einheiten der Formel (V)

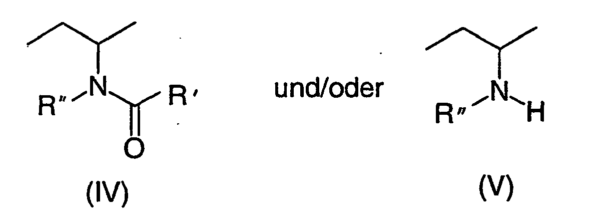

In den Formeln (IV) und (V) haben die Substituenten R' und R" jeweils die oben angegebene Bedeutung. In Abhängigkeit von den bei der Hydrolyse gewählten Reaktionsbedingungen enthält man entweder eine partielle oder vollständige Hydrolyse der Einheiten (IV).

Enthält die Pfropfgrundlage neben den hydrolyseunempfindlichen Vinylpyrrolidoneinheiten außerdem Comonomere, die hydrolyseempfindlich sind, wie z.B. Vinylacetat oder Acrylamid, so findet auch in der Pfropfgrundlage eine Hydrolyse statt. So reagiert Vinylacetat zu Vinylalkoholgruppen und Acrylamid zu Acrylsäuregruppen.

Als Hydrolysemittel eignen sich Mineralsäuren, wie Halogenwasserstoffe, die gasförmig oder in wässriger Lösung eingesetzt werden können. Vorzugsweise verwendet man Salzsäure, Schwefelsäure, Salpetersäure und Phosphorsäure sowie organische Säuren, wie C₁-bis C₅-Carbonsäuren und aliphatische oder aromatische Sulfonsäuren. Pro Formylgruppenäquivalent, das aus den einpolymerisierten Einheiten (IV) abgespalten werden soll, benötigt man 0,05 bis 2, vorzugsweise 1 bis 1,5 Moläquivalente einer Säure.

Die Hydrolyse der einpolymerisierten Einheiten der Struktur (IV) kann auch mit Hilfe von Basen vorgenommen werden, z.B. von Metallhydroxiden, insbesondere von Alkalimetall- und Erdalkalimetallhydroxiden. Vorzugsweise verwendet man Natriumhydroxid oder Kaliumhydroxid. Die Hydrolyse kann gegebenenfalls auch in Gegenwart von Ammoniak oder Aminen durchgeführt werden.

Die Hydrolyse im sauren oder im alkalischen pH-Bereich erfolgt z.B. bei Temperaturen von 30 bis 170, vorzugsweise 50 bis 120°C. Sie ist nach etwa 2 bis 8, vorzugsweise 3 bis 5 Stunden beendet. Nach diesen Reaktionszeiten erreicht man Hydrolysegrade der Einheiten der einpolymerisierten Monomeren der Formel (I) von 1 bis 100 %. Besonders bewährt hat sich eine Verfahrensweise, bei der zur Hydrolyse die Basen oder Säuren in wässriger Lösung zugesetzt werden. Nach der Hydrolyse führt man im allgemeinen eine Neutralisation durch, so dass der pH-Wert der hydrolysierten Polymerlösung 2 bis 8, vorzugsweise 3 bis 7 beträgt. Die Neutralisation ist dann erforderlich, wenn ein Fortschreiten der Hydrolyse von teilweise hydrolysierten Polymerisaten vermieden oder verzögert werden soll. Die Hydrolyse kann auch mit Hilfe von Enzymen vorgenommen werden.

Die so hergestellten Polymerisate können durch Umsetzung von im Polymer vorhandenen Hydroxyl- und/oder Aminofunktionen mit Epoxiden der Formel X nachträglich kationisiert werden (R³¹ = C₁ bis C₄₀ Alkyl).

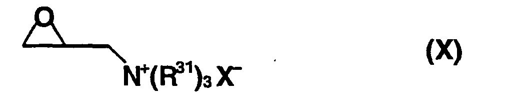

Dabei können bevorzugt die Hydroxylgruppen der Polyvinylalkohol-Einheiten und Vinylamin-Einheiten, entstanden durch Hydrolyse von Vinylformamid, mit den Epoxiden umgesetzt werden. Die Epoxide der Formel X können auch in situ durch Umsetzung der entsprechenden Chlorhydrine mit Basen, beispielsweise Natriumhydroxid, erzeugt werden.
Bevorzugt wird 2,3-Epoxypropyl-trimethylammoniumchlorid bzw. 3-Chlor-2-hydroxypropyl-trimethylammoniumchlorid eingesetzt.
Die K-Werte der Polymerisate sollen im Bereich von 10 bis 300, bevorzugt 25 bis 250, besonders bevorzugt 25 bis 200, ganz besonders bevorzugt im Bereich von 30 und 150, liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64, und 71 bis 74 (1932) in N-Methylpyrrolidon bei 25°C und Polymerkonzentrationen, die je nach K-Wert-Bereich zwischen 0,1 Gew.-% und 5 Gew.-% liegen.

Zur Entfernung von Lösungsmitteln können die Polymerlösungen wasserdampfdestilliert werden. Nach der Wasserdampfdestillation erhält man je nach Wahl der Komponenten wässrige Lösungen oder Dispersionen.

Die erhaltenen Polymerisate können auch nachträglich vernetzt werden, indem man die Hydroxylgruppen bzw. Aminogruppen im Polymer mit mindestens bifunktionellen Reagentien umsetzt. Bei niedrigen Vernetzungsgraden erhält man wasserlösliche Produkte, bei hohen Vernetzungsgrade wasserquellbare bzw. unlösliche Produkte.
Beispielsweise können die erfindungsgemäßen Polymerisate mit Dialdehyden und Diketonen, z.B. Glyoxal, Glutaraldehyd, Succindialdehyd oder Terephthalaldehyd, umgesetzt werden. Desweiteren eignen sich aliphatische oder aromatische Carbonsäuren, beispielsweise Maleinsäure, Oxalsäure, Malonsäure, Succinsäure oder Citronensäure, bzw. Carbonsäurederivaten wie Carbonsäureester, -anhydride oder -halogenide. Ferner sind mehrfunktionelle Epoxide geeignet, z.B.'Epichlorhydrin, Glycidylmethacrylat, Ethylenglykoldiglycidylether, 1,4-Butandioldiglycidylether oder 1,4-Bis(glycidyloxy)benzol. Ferner eigenen sich Diisocyanate, beispielsweise Hexamethylendiisocyanat, Isophorondiisocyanat, Methylendiphenyldiisocyanat, Toluylendiisocyanat oder Divinylsulfon.

Weiterhin eignen sich anorganische Verbindungen wie Borsäure oder Borsäuresalze, beispielsweise Natriummetaborat, Borax (Dinatriumtetraborat), sowie Salze mehrwertiger Kationen, z.B. Kupfer(II)-salze wie Kupfer(II)acetat oder Zink-, Aluminium-, Titansalze.

Borsäure bzw. Borsäuresalze wie Natriummetaborat oder Dinatriumtetraborat eignen sich bevorzugt zur nachträglichen Vernetzung. Dabei können die Borsäure bzw. Borsäuresalze, bevorzugt als Salzlösungen, den Lösungen der erfindungsgemäßen Polymerisate zugegeben werden. Bevorzugt werden die Borsäure bzw. Borsäuresalze den wässerigen Polymerisatlösungen hinzugefügt.

Die Borsäure bzw. Borsäuresalze können den Polymerlösungen direkt nach der Herstellung zugefügt werden. Es ist aber auch möglich, die Borsäure bzw. Borsäuresalze nachträglich den kosmetischen Formulierungen mit den erfindungsgemäßen Polymerisaten zuzusetzen, bzw. während des Herstellungsprozesses der kosmetischen Formulierungen.

Der Anteil Borsäure bzw. Borsäuresalze bezogen auf die erfindungsgemäßen Polymere beträgt 0 bis 15 Gew.-%, bevorzugt 0 bis 10 Gew.-%, besonders bevorzugt 0 bis 5 Gew.-%.

Die Polymerisatlösungen und -dispersionen können durch verschiedene Trocknungsverfahren wie z.B. Sprühtrocknung, Fluidized Spray Drying, Walzentrocknung oder Gefriertrocknung in Pulverform überführt werden. Als Trocknungsverfahren wird bevorzugt die Sprühtrocknung eingesetzt. Aus dem so erhaltenen Polymer-Trockenpulver läßt sich durch Lösen bzw. Redispergieren in Wasser erneut eine wäßrige Lösung bzw. Dispersion herstellen. Die Überführung in Pulverform hat den Vorteil einer besseren Lagerfähigkeit, einer einfacheren Transportmöglichkeit sowie eine geringere Neigung für Keimbefall.

Die erfindungsgemäßen wasserlöslichen oder wasserdispergierbaren Pfropfcopolymerisate hervorragend zur Verwendung in kosmetischen Formulierungen eignen, insbesondere als Verdicker.

Es wurde gefunden, dass die erfindungsgemäßen Polymere gute haarfestigende Eigenschaften aufweisen und gleichzeitig gute Gelbildner darstellen. Sie eignen sich somit insbesondere in verdickten kosmetischen Zubereitungen (z.B. Gele) und können in ihnen auch ohne den Zusatz üblicher Verdicker eingesetzt werden.

Die erfindungsgemäßen Polymere, eignen sich als Stylingmittel und/oder Konditioniermittel in haarkosmetischen Zubereitungen wie Haarkuren, Haarlotionen, Haarspülungen, Haaremulsionen, Spitzenfluids, Egalisierungsmittel für Dauerwellen, "Hot-Oil-Treatment"-Präparate, Conditioner, Festigerlotionen oder Haarsprays. Je nach Anwendungsbegiet können die haarkosmetischen Zubereitungen als Spray, Schaum, Gel, Gelspray oder Mousse appliziert werden.

Bevorzugt ist die Anwendung in Gel oder Gelspray.

Die erfindungsgemäßen haarkosmetischen Formulierungen enthalten in einer bevorzugten Ausführungsform
a) 0,05 - 20 Gew.-% des erfindungsgemäßen Polymeren
b) 20 - 99,95 Gew.-%Wasser und/oder Alkohol
c) 0 - 79,5 Gew.-%weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (Luviset^{®} P.U.R.) und Polyharnstoffe. Besonders geeignete Polymere sind Copolymere aus t-Butylacrylat, Ethylacrylat, Methacrylsäure (z.B. Luvimer^{®} 100P), Copolymere aus N-tert.-Butylacrylamid, Ethylacrylat, Acrylsäure (Ultrahold^{®} 8, strong), Copolymere aus Vinylacetat, Crotonsäure und gegebenenfall weiteren Vinylestern (z.B. Luviset^{®} Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol^{®} VBM).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCI, z.B. Copolymere aus Vinylpyrrolidon/N-Vinylimidazoliumsalzen (Luviquat^{®} FC, Luviquat^{®} HM, Luviquat^{®} MS, Luviquat^{®} Care), Copolymere aus N-Vinylpyrrolidon/Dimethylaminoethylmethacrylat, quaternisiert mit Diethylsulfat (Luviquat^{®} PQ 11), Copolymere aus N-Vinylcaprolactam N-Vinylpyrrolidon/N-Vinylimidazoliumsalzen (Luviquat^{®} Hold); kationische Cellulosederivate (Polyquaternium-4 und -10), Acrylamidcopolymere (Polyquaternium-7).

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymere eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas), besonders in Gelen und Gelsprays.

In einer bevorzugten Ausführungsform enthalten diese Zubereitungen
a) 0,1 - 10 Gew.-% des erfindungsgemäßen Polymeren
b) 20 - 99,9 Gew.-% Wasser und/oder Alkohol
c) 0 - 70 Gew.-% eines Treibmittel
d) 0 - 20 Gew.-% weitere Bestandteile

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält
a) 0,1 - 10 Gew.-% des erfindungsgemäßen Polymeren
b) 55 - 94,8 Gew.-% Wasser und/oder Alkohol
c) 5 - 20 Gew.-% eines Treibmittel
d) 0,1 - 5 Gew.-% eines Emulgators
e) 0 - 10 Gew.-% weitere Bestandteile

Als Emulgatoren können alle in Haarschäumen üblicherweise eingesetzten Emulgatoren verwendet werden. Geeignete Emulgatoren können nichtionisch, kationisch bzw. anionisch sein.
Beispiele für nichtionische Emulgatoren (INCI-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.
Beispiele für kationische Emulgatoren sind Cetyldimethyl-2-hydroxyethylammoniumdihydxogenphosphat, Cetyltrimoniumchlorid, Cetyltrimmoniumbromid, Cocotrimoniummethylsulfat, Quaternium-1 bis x (INCI).
Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Die erfindungsgemäßen Polymere können aufgrund ihrer verdickenden Wirkung als einziger Gelbildner in kosmetischen Zubereitungen eingesetzt werden. Darüberhinaus eignen sie sich auch zum Einsatz in Kombination mit üblichen Gelbildnern.

Überraschenderweise ist es möglich klare Gele zu erhalten.

Eine erfindungsgemäß für Styling-Gele geeignete Zubereitung kann beispielsweise wie folgt zusammengesetzt sein:
a) 0,1 - 10 Gew.-% des erfindungsgemäßen Polymeren
b) 60 - 99,85 Gew.-%Wasser und/oder Alkohol
c) 0,05 - 10 Gew.-%eines Gelbildners
d) 0 - 20 Gew.-% weitere Bestandteile

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Die erfindungsgemäßen Polymere können auch in Shampooformulierungen als Festigungs- und/oder Konditioniermittel eingesetzt werden.

Bevorzugte Shampooformulierungen enthalten
a) 0,05-20 Gew.-% des erfindungsgemäßen Polymeren
b) 25 - 94,95 Gew.-% Wasser
c) 5 - 50 Gew.-% Tenside
d) 0 - 5 Gew.-% eines weiteren Konditioniermittels
e) 0 - 10 Gew.-% weitere kosmetische Bestandteile

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.
Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.
Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Polymeren eingesetzt werden. Hierzu zählen beispielsweise kationische Polymere mit der Bezeichnung Polyquaternium nach INCI, insbesondere Copolymere aus Vinylpyrrolidon/N-Vinylimidazoliumsalzen (Luviquat^{®} FC, Luviquat^{®} HM, Luviquat^{®} MS, Luviquat^{®} Care), Copolymere aus N-Vinylpyrrolidon/Dimethylaminoethylmethacrylat, quaternisiert mit Diethylsulfat (Luviquat^{®} PQ 11), Copolymere aus N-Vinylcaprolactam/N-Vinylpyrrolidon/N-Vinylimidazoliumsalzen (Luviquat^{®} Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA).

### Beispiele:

### 1. Allgemeine Herstellvorschrift für Fällungspolymerisate Beispiele 1 bis 7

In einem gerührten Reaktor mit Stickstoffzuführung, Rückflußkühler und Dosiervorrichtung wird die Vorlage unter Stickstoff auf 87,5° erhitzt. Danach wird Zulauf 1 und 2 in 4 h, Zulauf 3 in 5 h (1/3 in 3 h 2/3 in 2 h) zudosiert. Anschließend wird auf 95°C aufheizt und 2 h bei bei dieser Temperatur auspolymerisiert. Nach Ende der Reaktion wir auf Raumtemperatur abgekühlt, mit Zulauf 4 verdünnt und das ausgefallene Polymer abfiltiert, mit Aceton gewaschen, trockengesaugt und im Vakuumtrockenschrank (Wasserstrahlpumpenvakuum) bei 75°C getrocknet.

Sämtliche Polymere werden in Ausbeuten > 95 % isoliert.

| Beispiel | 1 | 2 | 3 | 4 | 5 | 6 | 7 |
|---|---|---|---|---|---|---|---|
| Vorlage | | | | | | | |
| n-Butylacetat/Ethylacetat (Gew.Verhältnis 2:1) | 600 | 540 | 540 | 540 | 540 | 540 | 540 |
| Polyethylenglykol (M = 35 000) | 105,6 | 108 | 108 | 108 | 108 | 108 | 108 |
| Polyvinylethylether K-Wert (5 % in THF nach ISO1628-1): 10-17 (40 %ige Lösung in Butylacetat) | 13,2 | 13,5 | 13,5 | 13,5 | 13,5 | 13,5 | 13,5 |
| Pentaerythrittriallylether (techn. Aldrich) | 1,48 | 2,16 | | 2,16 | | 2,16 | 2,16 |
| Zulauf 3 | 8 | 7 | 7 | 7 | 7 | 7 | 7 |
| | | | | | | | |

| Zulauf 1 | | | | | | | |
|---|---|---|---|---|---|---|---|
| n-Butylacetat/Ethylacetat | 197,12 | 99 | 99 | 99 | 99 | 76,5 | 96 |
| N-Vinylformamid | 253,44 | 270 | 270 | 270 | 270 | 202,5 | 288 |
| Stearylmethacrylat | 42,24 | 27 | 27 | 27 | 27 | 27 | |
| | | | | | | | |
| Zulauf 2 | | | | | | | |
| n-Butylacetat/Ethylacetat (Gew.Verhältnis 2:1) | 84,48 | 135 | 135 | 135 | 135 | 202,5 | 144 |
| Acrylsäure (AS) | 126,72 | 135 | 135 | 135 | 135 | 202,5 | 144 |
| | | | | | | | |

| Zulauf 3 | | | | | | | |
|---|---|---|---|---|---|---|---|
| n-Butylacetat/Ethylacetat | 156,83 | 156,76 | 156,76 | 158,38 | 158,38 | 156,76 | 156,76 |
| Tert-Butylperoctoat | 3,17 | 3,24 | 3,24 | 1,62 | 1,62 | 3,24 | 3,24 |
| | | | | | | | |

| Zulauf 4 | | | | | | | |
|---|---|---|---|---|---|---|---|
| Aceton | 500 | 500 | 500 | 500 | 500 | 500 | 500 |

### 2. Herstellvorschrift der wässrigen, mit AMP neutralisierten Lösungen

8 g Polymer (jeweils aus den Beispielen 1 bis 7) werden in einem Planschliffreaktionskolben mit Ankerrührer, Innenthermometer, Kühler und Ölbad vorgelegt, mit 0,1 % bez. auf Gesamtansatz Konservierungsmittel Euxyl K 100 (Lösung von Isothiazolinonderivaten und Benzylalkohol, der Firma Schülke & Mayr) versetzt, mit 400 g Wasser versetzt und unter Rühren auf 80°C aufgeheizt. Die berechnete Basenmenge 2-Amino-2-methylpropanol-1 (AMP) wird mit 200 g Wassers gemischt und zu der Polymersuspension zugeben und solange bei 80°C rühren bis das Polymer gelöst ist. Anschließend wird die Restmenge Wasser für einen Gesamtansatz von 800 g eingerührt und die warme Lösung abgefüllt.

### 3. Viskositätsbestimmung

Die Viskosität wurde nach Brookfield bei 25°C bestimmt (Brookfieldviskosimeter LVDV-II+).

| Beispiel Nr. | AMP | | Viskosität | |
|---|---|---|---|---|
| | (10 %ig in Wasser) in g | Spindel | Drehzahl | CP |
| 1 | | 3 | 6 | 12897 |
| 2 | 24,4 | 4 | 30 | 12257 |
| 3 | 24,5 | 1 | 30 | 44,4 |
| 4 | 24,4 | 4 | 6 | 68278 |
| 5 | 24,5 | 1 | 30 | 23,4 |
| 6 | 36,6 | 4 | 30 | 7138 |
| 7 | 26,1 | 4 | 30 | 6259 |

### 4. Carbopolverträglichkeit:

Einsatzstoffe: Mit AMP-neutralsierte Lösung der Polymere gemäß obiger Vorschrift, Carbopollösung 1 %ig (Carbopol 940), Triethanolamin.

### Durchführung:

100 g der Polymlösung werden in eine 250 ml Weithalsglasflasche abwogen und 98,68 g einer 1 %igen wässrigen Lösung von Carbopol 940 zugegeben und gut vermischt. Anschließend werden 1,32 g Triethanolamin tropfenweise zugeben, wiederum gut vermischt und das erhaltene Gel oder die Lösung beurteilen.

| Beispiel Nr. | AMP-neutralisierte Polymerlösung (s. unter 2) | Gel: neutralisierte Polymerlösung mit Carbolösung (siehe unter 4) |
|---|---|---|
| 1 | - | ++ |
| 2 | ++ | ++ |
| 4 | ++ | ++ |
| 6 | - | ++ |
| 7 | - | ++ |

### Bewertung:

fast klare Lösung (+++) > leicht trübe Lösung (++) > etwas trübe Lösung (+) > trübe Lösung (-)

### 5. Festigungswirkung

Ansatz.: 50 g fertiges Gel (1 %ig mit AMP neutralisiert) 170 g Wasser dest.
Feststoffgehalt von ca. 0,23 %.

### Biegetest

Die trockenen, gewogenen Haarsträhnen werden 3 x durch das verdünnte Gel durchgezogen. Der Überschuß wird nach jedem Durchziehen mit den Fingern abgestrichen. Nach dem letzten Benetzen wird die Haarsträhne zwischen Filterpapier abgedrückt. Die feuchte Haarsträhne wird gewogen und dann so geformt, daß es einen runden Durchschnitt ergibt. Die Auftragsmenge beträgt zwischen 1,00 bis 1,30 g (abhängig von der Viskosität). Nach dem Trocknen über Nacht im Klimaraum (20°C, 65 % rel. Feuchte). wird mit der Zug-Druck-Maschine (Fa.. Franck) der Biegetestwert gemessen.

### Curl retention:

Die trockene Haarsträhne wird 3x durch das verdünnte Gel durchgezogen. Der Überschuß wird nach jedem Durchziehen mit den Fingern abgestrichen. Die gleichmäßig feuchte Haarsträhne wird nach dem letzten Benetzen gekämmt und auf Teflonstäbe (Ø 12 mm) gewikkelt. Zwischendurch wird die Haarsträhne immer wieder gekämmt. Die Haarsträhne wird mit einem Filterpapierstreifen und Gummiringen befestigt. Die Wickel werden über Nacht bei 70°C getrocknet. Nach dem Abkühlen auf RT werden das Papier und die Gummiringe entfernt und die Haarsträhne vorsichtig von dem Teflonstab angegestreift.

Die Ausgangslänge und die Länge nach 5 Stunden Klimabehandlung wird bestimmt und die Curl retention berechnet.

| Beispiel Nr. | Biegetest (cN) | Curl-Retention (%) |
|---|---|---|
| 2 | 88 | 91 |
| 4 | 107 | 85 |
| 7 | 120 | 90 |

### Vergleich:

| Polymere | Biegetest (cN) | Curl-retention (%) |
|---|---|---|
| Vinylpyrrolidon/Vinylacetat Copolymer erhältlich unter dem Handelsnamen Luviskol VA64 P | 59 | 31 |
| Polyvinylpyrrolidone erhältlich unter dem Handelsnamen Luviskol K30 | 74 | 27 |

### Anwendungsbeispiele

Die folgenden Formulierungen wurden jeweils mit den 7 erfindungsgemäßen Polymeren hergestellt.

Beispiele 1 bis 7: Formulierung Aerosolhaarschaum:
2,00 % Copolymer aus Beispiel 1 bis 7
2,00 % Luviquat Mono LS (Coco trimonium methyl sulfat)
67,7 % Wasser
10,0 Propan/Butan 3,5 bar (20°C)
q.s. Parfümöl

### Vergleichsbeispiel:

2,00 % Polymergehalt Luviquat Hold (Polquaternium-46)
2,00 % Luviquat Mono LS (Coco trimonium methyl sulfat)
67,7 % Wasser
10,0 Propan/Butan 3,5 bar (20°C)
q.s. Parfümöl

### Beispiel 8 bis 14: Aerosolhaarschaum:

| | INCI |
|---|---|
| 4,00 % Copolymer aus Beispiel 1 bis 7 | |
| 0,20 % Cremophor A 25 | Ceteareth-25 |
| 1,00 % Luviquat Mono CP | Hydroxyethyl cetyldi- |
| | monium phosphate |
| 5,00 % Ethanol | |
| 1,00 % Panthenol | |
| 10,0 Propan/Butan 3,5 bar (20°C) | |
| q.s. Parfümöl | |
| ad 100 % Wasser | |

### Beispiele 15 bis 21: Pumpschäume:

| | INCI |
|---|---|
| 2,00 % Copolymer aus Beispiel 1 bis 7 | |
| 2,00 % Luviflex Soft (Polymergehalt) | |
| 1,20 % 2-Amino-2-methyl-1-propanol | |
| 0,20 % Cremophor A 25 | |
| 0,10 % Uvinul P 25 | PEG-25 PABA |
| q.s. Konservierungsmittel | |
| q.s. Parfümöl | |
| ad 100 % Wasser | |

### Beispiele 22 bis 28: Pumpsprays

| | INCI |
|---|---|
| 4,00 % Copolymer aus Beispiel 1 bis 7 | |
| 1,00 % Panthenol | |
| 0,10 % Uvinul MS 40 | Benzophenone-4 |
| q.s. Konservierungsmittel | |
| q.s. Parfümöl | |
| ad 100 % Wasser | |

### Beispiele 29 bis 35: Pumpsprays:

| | INCI |
|---|---|
| 4,00 % Copolymer aus Beispiel 1 bis 7 | |
| 1,00 % Panthenol | |
| 0,10 % Uvinul M 40 | Benzophenone-3 |
| q.s. Konservierungsmittel | |
| q.s. Parfümöl | |
| ad 100 % Ethanol | |

### Beispiele 36 bis 42: Haarsprays:

| | INCI |
|---|---|
| 5,00 % Copolymer aus Beispiel 1 bis 7 | |
| 0,10 % Siliconöl Dow Corning DC 190 | Dimethicone Copolyol |
| 35,00 % Dimethylether | |
| 5,00 % n-Pentan | |
| ad 100 % Ethanol | |
| q.s. Parfümöl | |

### Beispiele 43 bis 49: Haarsprays VOC 55 %:

| | INCI |
|---|---|
| 3,00 % Copolymer aus Beispiel 1 bis 7 | |
| 7,00 % Luviset P.U.R. | Polyurethane-1 |
| 40,00 % Dimethylether | |
| 15,00 % Ethanol | |
| q.s. Parfümöl | |
| ad 100 % Wasser | |

### Beispiele 50 bis 56: Haargele:

| | INCI |
|---|---|
| 0,50 % Carbopol 980 | Cabomer |
| 3,00 % Copolymer aus Beispiel 1 bis 7 | |
| 0,10 % Phythantriol | |
| 0,50 % Panthenol | |
| q.s. Parfümöl | |
| q.s Konservierungsmittel | |
| ad 100 % Wasser | |

### Beispiele 57 bis 64: Haarshampoo bzw. Duschgel

| | INCI |
|---|---|
| 0,50 % Copolymer aus Beispiel 1 bis 7 | |
| 40,00 % Texapon NSO | Sodium Laureth Sulfate |
| 5,00 % Tego Betain L 7 | Cocamidopropyl Betaine |
| 5,00 % Plantacare 2000 | Decyl Glucoside |
| 1,00 % Propylenglycol | |
| q.s. Citronensäure | |
| q.s. Konservierungsmittel | |
| 1,00 % Natriumchlorid | |
| ad 100 % Wasser | |

### Anwendungsbeispiele 65 bis 71: Hautcreme

Gemäß folgender Rezeptur wurden Wasser/Öl-Cremeemulsionen hergestellt:

| | Zusatz | Gew.-% |
|---|---|---|
| Cremophor A 6 | Ceteareth-6 und Stearylalkohol | 2,0 |
| Chremophor A 25 | Ceteareth-25 | 2,0 |
| Lanette O | Cetearylalkohol | 2,0 |
| Imwitor 960 K | Glycerylstearat SE | 3,0 |
| Paraffinöl | | 5,0 |
| Jojobaöl | | 4,0 |
| Luvitol EHO | Cetearyloctanoat | 3,0 |
| ABIL 350 | Dimethicone | 1,0 |
| Amerchol L 101 | Mineralöl und Lanolinalkohol | 3,0 |
| Veegum Ultra | Magnesiumaluminiumsilikat | 0,5 |
| 1,2-Propylenglykol | Propylenglykol | 5,0 |
| Abiol | Imidazolindinyl-Harnstoff | 0,3 |
| Phenoxyethanol | | 0,5 |
| D-Panthenol USP | | 1,0 |
| Polymer (Herstellungsbeispiel 1 bis 7) | | 0,5 |
| Wasser | | ad 100 |

### Anwendungsbeispiele 72 bis 78: Duschgele

Gemäß folgender Rezeptur wurden Duschgel-Formulierungen hergestellt:

| | Zusatz | Gew.-% |
|---|---|---|
| Texapon NSO | Natriumlaurethsulfat | 40,0 |
| Tego Betain L7 | Cocamidopropylbetain | 5,0 |
| Plantacare 2000 | Decylglucosid | 5,0 |
| Parfüm | | 0,2 |
| Polymer gemäß Herstellungsbeispiel 1 bis 7 | | 0,2 |
| Euxyl K 100 | Benzylalkohol, Methylchlorisothiazolinon, Methylisothiazolinon | 0,1 |
| D-Panthenol USP | | 0,5 |
| Citronensäure (pH 6-7) | | q.s. |
| NaCl | | 2,0 |
| Wasser | | ad 100 |

### Anwendungsbeispiele: Feuchthalteformulierungen

### Formulierung A

| | | Zusatz | Gew.-% |
|---|---|---|---|
| a) | Cremophor A6 | Ceteareth-6 und Stearylalkohol | 2,0 |
| | Cremophor A25 | Ceteareth-25 | 2,0 |
| | Paraffinöl (dickflüssig) | | 10 |
| | Lannette O | Cetearylalkohol | 2,0 |
| | Stearinsäure | | 3,0 |
| | Nip-Nip | Methylparaben/Propylparaben 70:30 | 0,5 |
| | Abiol | Imidazoldinyl-Harnstoff | 0,5 |
| b) | Polymer (Herstellungsbeispiel 1 bis 7) | | 3,0 |
| | Wasser | | ad 100,0 |

Beide Phasen wurden auf 80°C erhitzt, Phase a) wurde in b) eingerührt, homogenisiert und kaltgerührt und anschließend mit 10%iger wässriger NaOH-Lösung auf pH 6 eingestellt.

### Anwendungsbeispiele: O/W Cremes zur Hautfeuchthaltung

| Zusatz | Gew.-% |
|---|---|
| Glycerinmonostearat | 2,0 |
| Cetylalkohol | 3,0 |
| Paraffinöl, subliquidum | 15,0 |
| Vaseline | 3,0 |
| Caprylcaprinsäuretriglycerid | 4,0 |
| Octyldodecanol | 2,0 |
| Hydriertes Kokosfett | 2,0 |
| Cetylphosphat | 0,4 |
| Polymer (Herstellungsbeispiel 1 bis 7) | 3,0 |
| Glycerin | 3,0 |
| Natriumhydroxid | q.s. |
| Parfümöl | q.s. |
| Konservierungsmittel | q.s. |
| Wasser | ad 100 |

### Anwendungsbeispiele: O/W-Lotionen

| Zusatz | Gew.-% |
|---|---|
| Stearinsäure | 1,5 |
| Sorbitanmonostearat | 1,0 |
| Sorbitanmonooleat | 1,0 |
| Paraffinöl, subliquidum | 7,0 |
| Cetylalkohol | 1,0 |
| Polydimethylsiloxan | 1,5 |
| Glycerin | 3,0 |
| Polymer (Herstellungsbeispiel 1 bis 7) | 0,5 |
| Parfümöl | q.s. |
| Konservierungsmittel | q.s. |
| Wasser | ad 100 |

### Anwendungsbeispiele: W/O-Cremes

| Zusatz | Gew.-% |
|---|---|
| PEG-7-hydriertes Ricinusöl | 4,0 |
| Wollwachsalkohol | 1,5 |
| Bienenwachs | 3,0 |
| Triglycerid, flüssig | 5,0 |
| Vaseline | 9,0 |
| Ozokerite | 4,0 |
| Paraffinöl, subliquidum | 4,0 |
| Glycerin | 2,0 |
| Polymer (Herstellungsbeispiel 1 bis 7) | 2,0 |
| Magnesiumsulfat*7H₂O | 0,7 |
| Parfümöl | q.s. |
| Konservierungsmittel | q.s. |
| Wasser | ad 100 |

### Anwendungsbeispiele: Hydrogele zur Hautpflege

| Zusatz | Gew.-% |
|---|---|
| Polymer (Herstellungsbeispiel 1 bis 7) | 3,0 |
| Sorbit | 2,0 |
| Glycerin | 3,0 |
| Polyethylenglycol 400 | 5,0 |
| Ethanol | 1,0 |
| Parfümöl | q.s. |
| Konservierungsmittel | q.s. |
| Wasser | ad 100 |

### Anwendungsbeispiele: Hydrodispersionsgele

| Zusatz | Gew.-% |
|---|---|
| Polymer (Herstellungsbeispiel 1 bis 7) | 3,0 |
| Sorbit | 2,0 |
| Glycerin | 3,0 |
| Polyethylenglycol 400 | 5,0 |
| Triglycerid, flüssig | 2,0 |
| Ethanol | 1,0 |
| Parfümöl | q.s. |
| Konservierungsmittel | q.s. |
| Wasser | ad 100 |

### Anwendungsbeispiele: Flüssige Seifen

| Zusatz | Gew.-% |
|---|---|
| Kokosfettsäure, Kaliumsalz | 15 |
| Kaliumoleat | 3 |
| Glycerin | 5 |
| Polymer (Herstellungsbeispiel 1 bis 7) | 2 |
| Glycerinstearat | 1 |
| Ethylenglycoldistearat | 2 |
| Spezifische Zusätze, Komplexierungsmittel, Duftstoffe | q.s. |
| Wasser | ad 100 |

### Anwendungsbeispiele: Body Care Creams

| | Zusatz | Gew.-% |
|---|---|---|
| Cremophor A6 | Ceteareth-6 und Stearylalkohol | 2,0% |
| Cremophor A 25 | Ceteareth-25 | 2,0% |
| Grape (Vitis Vinifera)Seed oil | | 6,0% |
| Glycerylstearat SE | | 3,0% |
| Cetearylalkohol | | 2,0% |
| Dimethicon | | 0,5% |
| Luvitol EHO | Cetearyloctanoat | 8,0% |
| Oxynex 2004 | Propylenglycol, BHT, Ascorbylpalmitat, Glycerylstearat, Citronensäure | 0,1% |
| Konservierungsmittel | | q.s. |
| 1,2-Propylenglykol USP | | 3,0% |
| Glycerin | | 2,0% |
| EDTA BD | | 0,1% |
| D-Panthenol USP | | 1,0% |
| Wasser | | ad 100 |
| Polymer (Herstellungsbeispiel 1 bis 7) | | 1,5% |
| Tocopherylacetat | | 0,5% |

In den folgenden Anwendungsbeispielen sind alle Mengenangaben in Gew.-%.

### Anwendungsbeispiele: Flüssige Makeups

| A | |
|---|---|
| 1,70 | Glyceryl Stearate |
| 1,70 | Cetyl Alcohol |
| 1,70 | Ceteareth-6 |
| 1,70 | Ceteareth-25 |
| 5,20 | Caprylic/Capric Triglyceride |
| 5,20 | Mineral Oil |

| B | |
|---|---|
| q.s. | Konservierungsmittel |
| 4,30 | Propylene Glycol |
| 2,50 | Polymerisat gemäß Herstellbeispiel 1 bis 7 |
| 59,50 | dest. Wasser |

| C | |
|---|---|
| q.s. | Parfumöl |

| D | |
|---|---|
| 2,00 | Iron Oxides |
| 12,00 | Titanium Dioxide |

### Herstellung:

Phase A und Phase B getrennt voneinunder auf 80°C erwärmen. Dann Phase B in Phase A mit einem Rührer mischen. Alles auf 40°C abkühlen lassen und Phase C und Phase D zugeben. Wiederholt homogenisieren.

### Anwendungsbeispiele: Ölfreie Makeups

| A | |
|---|---|
| 0,35 | Veegum |
| 5,00 | Butylene Glykol |
| 0,15 | Xanthan Gum |

| B | |
|---|---|
| 53,0 | dest. Wasser |
| q.s. | Konservierungsmittel |
| 0,2 | Polysorbate-20 |
| 1,6 | Tetrahydroxypropyl Ethylenediamine |

| C | |
|---|---|
| 1,0 | Silica |
| 2,0 | Nylone-12 |
| 4,15 | Mica |
| 6,0 | Titanium Dioxide |
| 1,85 | Iron Oxides |

| D | |
|---|---|
| 4,0 | Stearic Acid |
| 1,5 | Glyceryl Stearate |
| 7,0 | Benzyl Laurate |
| 5,0 | Isoeicosane |
| q.s. | Konservierungsmittel |

| E | |
|---|---|
| 1,0 | dest. Wasser |
| 0,5 | Panthenol |
| 0,1 | Imidazolidinyl Urea |
| 5,0 | Polymerisat gemäß Herstellbeispiel 1 bis 7 |

### Herstellung:

Phase A mit Butylene Glykol benetzen, in Phase B hineingeben und gut mischen. Phase AB auf 75°C erwärmen. Phase C Einsatzstoffe pulverisieren, in Phase AB hineingeben und gut homogenisieren. Einsatzstoffe von Phase D mischen, auf 80°C erwärmen und zu Phase ABC geben. Einige Zeit mischen, bis alles homogen ist. Alles in ein Gefäß mit Propellermischer übertragen. Einsatzstoffe von Phase E mischen, in Phase ABCD hineingeben und gut vermischen.

### Anwendungsbeispiele: Eyeliner

| A | |
|---|---|
| 40,6 | dest. Wasser |
| 0,2 | Disodium EDTA |
| q.s. | Konservierungsmittel |

| B | |
|---|---|
| 0,6 | Xanthan Gum |
| 0,4 | Veegum |
| 3,0 | Butylene Glycol |
| 0,2 | Polysorbate-20 |

| C | |
|---|---|
| 15,0 | Iron oxide / Al Powder / Silica (z.B. Sicopearl Fantastico |
| | Gold ^{™} von BASF) |

| D | |
|---|---|
| 10,0 | Dest. Wasser |
| 30,0 | Polymerisat gemäß Herstellbeispiel 1 bis 7 |

### Herstellung:

Phase B vormischen. Mit einem Propellermischer Phase B in Phase A hineinmischen, wobei man den Verdicker quellen lässt. Phase C mit Phase D benetzen, alles in Phases AB zugeben und gut mischen.

### Anwendungsbeispiele: Schimmernde Gele

| A | |
|---|---|
| 32,6 | Dest. Wasser |
| 0,1 | Disodium EDTA |
| 25,0 | Carbomer (2%ige wässrige Lösung) |
| 0,3 | Konservierungsmittel |

| B | |
|---|---|
| 0,5 | Dest. Wasser |
| 0,5 | Triethanolamine |

| C | |
|---|---|
| 10,0 | Dest. Wasser |
| 9,0 | Polymerisat gemäß Herstellbeispiel 1 bis 7 |
| 1,0 | Polyquaternium-46 |
| 5,0 | Iron Oxide |

| D | |
|---|---|
| 15,0 | Dest. Wasser |
| 1,0 | D-Panthenol 50 P (Panthenol und Propylene Glycol) |

### Herstellung:

Mit einem Propellermischer die Einsatzstoffe von Phase A in der angegebenen Reihenfolge gut mischen. Dann Phase B in Phase A hineingeben. Langsam rühren bis alles homogen ist. Phase C gut homogenisieren, bis die Pigmente gut verteilt sind. Phase C und Phase D zu Phase AB geben und gut mischen.

### Anwendungsbeispiele: Wasserfeste Mascaras

| A | |
|---|---|
| 46,7 | Dest. Wasser |
| 3,0 | Lutrol E 400 (PEG-8) |
| 0,5 | Xanthan Gum |
| q.s. | Konservierungsmittel |
| 0,1 | Imidazolidinyl Urea |
| 1,3 | Tetrahydroxypropyl Ethylenediamine |

| B | |
|---|---|
| 8,0 | Carnauba Wax |
| 4,0 | Beeswax |
| 4,0 | Isoeicosane |
| 4,0 | Polyisobutene |
| 5,0 | Stearic Acid |
| 1,0 | Glyceryl Stearate |
| q.s. | Konservierungsmittel |
| 2,0 | Benzyl Laurate |

| C | |
|---|---|
| 10,0 | Iron oxide / Al Powder / Silica (z.B. Sicopearl Fantastico |
| | Gold ^{™} von BASF) |

| E | |
|---|---|
| 8,0 | Polyurethane-1 |
| 2,0 | Polymerisat gemäß Herstellbeispiel-1 bis 7 |

### Herstellung:

Phase A und Phase B getrennt voneinander auf 85ºC erwärmen. Temperatur halten und Phase C zu Phase A geben und homogenisieren, bis die Pigmente gleichmäßig verteilt sind. Phase B zu Phases AC geben und für 2-3 Minuten homogenisieren. Dann Phase E zugeben und langsam rühren. Alles auf Raumtemperatur abkühlen lassen.

### Anwendungsbeispiele: Sonnenschutz-Gele

| Phase A | |
|---|---|
| 1,00 | PEG-40 Hydrogenated Castor Oil |
| 8,00 | Octyl Methoxycinnamate (Uvinul MC 80^{™} von BASF) |
| 5,00 | Octocrylene (Uvinul N 539^{™} von BASF) |
| 0,80 | Octyl Triazone (Uvinul T 150 ^{™} von BASF) |
| 2,00 | Butyl Methoxydibenzoylmethane (Uvinul BMBM^{™} von BASF) |
| 2,00 | Tocopheryl Acetate |
| q.s. | Parfümöl |

| Phase B | |
|---|---|
| 2,50 | Polymerisat gemäß Herstellbeispiel 1 bis 7 |
| 0,30 | Acrylates/C10-30 Alkyl Acrylate Crosspolymer |
| 0,20 | Carbomer |
| 5,00 | Glycerin |
| 0,20 | Disodium EDTA |
| q.s. | Konservierungsmittel |
| 72,80 | dest. Wasser |

| Phase C | |
|---|---|
| 0,20 | Sodium Hydroxide |

### Herstellung:

Die Komponenten der Phase A mischen. Phase B quellen lassen und unter Homogenisieren in Phase A einrühren. Mit Phase C neutralisieren und erneut homogenisieren.

### Anwendungsbeispiele: Sonnenschutzemulsionen mit TiO₂ und ZnO₂

| Phase A | |
|---|---|
| 6,00 | PEG-7 Hydrogenated Castor Oil |
| 2,00 | PEG-45/Dodecyl Glycol Copolymer |
| 3,00 | Isopropyl Myristate |
| 8,00 | Jojoba (Buxus Chinensis) Oil |
| 4,00 | Octyl Methoxycinnamate (Uvinul MC 80) |
| 2,00 | 4-Methylbenzylidene Camphor (Uvinul MBC 95) |
| 3,00 | Titanium Dioxide, Dimethicone |
| 1,00 | Dimethicone |
| 5,00 | Zinc Oxide, Dimethicone |

| Phase B | |
|---|---|
| 2,00 | Polymerisat gemäß Herstellbeispiel 1 bis 7 |
| 0,20 | Disodium EDTA |
| 5,00 | Glycerin |
| q.s. | Konservierungsmittel |
| 58,80 | dest. Wasser |

| Phase C | |
|---|---|
| q.s. | Parfümöl |

### Herstellung:

Die Phasen A und B getrennt auf ca. 85°C erwärmen. Phase B in Phase A einrühren und homogenisieren. Abkühlen auf ca. 40°C, Phase C hinzugeben und nochmals kurz homogenisieren.

### Anwendungsbeispiele: Sun Protection Lotions

| Phase A | |
|---|---|
| 6,00 | Octyl Methoxycinnamate (Uvinul MC 80 ^{™} von BASF) |
| 2,50 | 4-Methylbenzylidene Camphor (Uvinul MBC 95 ^{™} von BASF) |
| 1,00 | Octyl Triazone (Uvinul T 150 ^{™} von BASF) |
| 2,00 | Butyl Methoxydibenzoylmethane (Uvinul BMBM ^{™} von BASF) |
| 2,00 | PVP/Hexadecene Copolymer |
| 5,00 | PPG-3 Myristyl Ether |
| 0,50 | Dimethicone |
| 0,10 | BHT, Ascorbyl Palmitate, Citric Acid, Glyceryl Stearate, |
| | Propylene Glycol |
| 2,00 | Cetyl Alcohol |
| 2,00 | Potassium Cetyl Phosphate |

| Phase B | |
|---|---|
| 2,50 | Polymerisat gemäß Herstellbeispiel 1 bis 7 |
| 5,00 | Propylene Glycol |
| 0,20 | Disodium EDTA |
| q.s. | Konservierungsmittel |
| 63,92 | dest. Wasser |

| Phase C | |
|---|---|
| 5,00 | Mineral Oil |
| 0,20 | Carbomer |

| Phase D | |
|---|---|
| 0,08 | Sodium Hydroxide |

| Phase E | |
|---|---|
| q.s. | Parfümöl |

### Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

### Anwendungsbeispiele: Abziehbare Gesichtsmasken

| Phase A | |
|---|---|
| 57,10 | dest. Wasser |
| 6,00 | Polyvinyl Alcohol |
| 5,00 | Propylene Glycol |

| Phase B | |
|---|---|
| 20,00 | Alcohol |
| 4,00 | PEG-32 |
| q.s | Parfümöl |

| Phase C | |
|---|---|
| 5,00 | Polyquaternium-44 |
| 2,70 | Polymerisat gemäß Herstellbeispiel 1 bis 7 |
| 0,20 | Allantoin |

### Herstellung:

Phase A auf mind. 90°C erwärmen und rühren bis gelöst. Phase B bei 50°C lösen und in Phase A einrühren. Bei ca. 35°C den Ethanolverlust ausgleichen. Phase C zugeben und unterrühren.

### Anwendungsbeispiele: Gesichtsmasken

| Phase A | |
|---|---|
| 3,00 | Ceteareth-6 |
| 1,50 | Ceteareth-25 |
| 5,00 | Cetearyl Alcohol |
| 6,00 | Cetearyl Octanoate |
| 6,00 | Mineral Oil |
| 0,20 | Bisabolol |
| 3,00 | Glyceryl Stearate |

| Phase B | |
|---|---|
| 2,00 | Propylene Glycol |
| 5,00 | Panthenol |
| 2,80 | Polymerisat gemäß Herstellbeispiel 1 bis 7 |
| q.s. | Konservierungsmittel |
| 65,00 | dest. Wasser |

| Phase C | |
|---|---|
| q.s. | Parfümöl |
| 0,50 | Tocopheryl Acetate |

### Herstellung:

Phase A und B getrennt auf ca. 80°C erwärmen. Phase B in Phase A unter Homogenisieren einrühren, kurz nachhomogenisieren. Abkühlen auf ca. 40°C, Phase C hinzugeben, nochmals homogenisieren.

### Anwendungsbeispiele: Körperlotion-Schäume

| Phase A | |
|---|---|
| 1,50 | Ceteareth-25 |
| 1,50 | Ceteareth-6 |
| 4,00 | Cetearyl Alcohol |
| 10,00 | Cetearyl Octanoate |
| 1,00 | Dimethicone |

| Phase B | |
|---|---|
| 3,00 | Polymerisat gemäß Herstellbeispiel 1 bis 7 |
| 2,00 | Panthenol |
| 2,50 | Propylene Glycol |
| q.s. | Konservierungsmittel |
| 74,50 | dest. Wasser |

| Phase C | |
|---|---|
| q.s. | Parfümöl |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Abfüllung: 90 % Wirkstoff und 10 % Propan/Butan bei 3,5 bar (20°C).

Anwendungsbeispiele: Gesichtswasser für trockene und empfindliche Haut

| Phase A | |
|---|---|
| 2,50 | PEG-40 Hydrogenated Castor Oil |
| q.s. | Parfümöl |
| 0,40 | Bisabolol |

| Phase B | |
|---|---|
| 3,00 | Glycerin |
| 1,00 | Hydroxyethyl Cetyldimonium Phosphate |
| 5,00 | Whitch Hazel (Hamamelis Virginiana) Distillate |
| 0,50 | Panthenol |
| 0,50 | Polymerisat gemäß Herstellbeispiel 1 bis 7 |
| q.s. | Konservierungsmittel |
| 87,60 | dest. Wasser |

### Herstellung:

Phase A klar lösen. Phase B in Phase A einrühren.

### Anwendungsbeispiele: Gesichtswaschpasten mit Peelingeffekt

| Phase A | |
|---|---|
| 70,00 | dest. Wasser |
| 3,00 | Polymerisat gemäß Herstellbeispiel 1 bis 7 |
| 1,50 | Carbomer |
| q.s. | Konservierungsmittel |

| Phase B | |
|---|---|
| q.s. | Parfümöl |
| 7,00 | Potassium Cocoyl Hydrolyzed Protein |
| 4,00 | Cocamidopropyl Betaine |

| Phase C | |
|---|---|
| 1,50 | Triethanolamine |

| Phase D | |
|---|---|
| 13,00 | Polyethylene (Luwax A^{™} von BASF) |

### Herstellung:

Phase A quellen lassen. Phase B klar lösen. Phase B in Phase A einrühren. Mit Phase C neutralisieren. Danach Phase D einrühren.

### Anwendungsbeispiele: Gesichtsseifen

| Phase A | |
|---|---|
| 25.0 | Potassium Cocoate |
| 20.0 | Disodium Cocoamphodiacetate |
| 2.0 | Lauramide DEA |
| 1.0 | Glycol Stearate |
| 2.0 | Polymerisat gemäß Herstellbeispiel 1 bis 7 |
| 50.0 | dest. Wasser |
| q.s. | Citric Acid |

| Phase B | |
|---|---|
| q.s. | Konservierungsmittel |
| q.s. | Parfumöl |

### Herstellung:

Phase A unter Rühren auf 70°C erwärmen, bis alles homogen ist. pH-Wert auf 7.0 bis 7.5 mit Citric Acid. Alles auf 50°C abkühlen lassen und Phase B zugeben.

### Anwendungsbeispiele: Gesichtsreinigungsmilch Typ O/W

| Phase A | |
|---|---|
| 1,50 | Ceteareth-6 |
| 1,50 | Ceteareth-25 |
| 2,00 | Glyceryl Stearate |
| 2,00 | Cetyl Alcohol |
| 10,00 | Mineral Oil |

| Phase B | |
|---|---|
| 5,00 | Propylene Glycol |
| q.s. | Konservierungsmittel |
| 1,0 | Polymerisat gemäß Herstellbeispiel 1 bis 7 |
| 66,30 | dest. Wasser |

| Phase C | |
|---|---|
| 0,20 | Carbomer |
| 10,00 | Cetearyl Octanoate |

| Phase D | |
|---|---|
| 0,40 | Tetrahydroxypropyl Ethylenediamine |

| Phase E | |
|---|---|
| q.s. | Parfümöl |
| 0,10 | Bisabolol |

### Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

### Anwendungsbeispiele: Transparente Seifen

| |
|---|
| 4,20 Sodium Hydroxide |
| 3,60 dest. Wasser |
| 2,0 Polymerisat gemäß Herstellbeispiel 1 bis 7 |
| 22,60 Propylene Glycol |
| 18,70 Glycerin |
| 5,20 Cocoamide DEA |
| 10,40 Cocamine Oxide |
| 4,20 Sodium Lauryl Sulfate |
| 7,30 Myristic Acid |
| 16,60 Stearic Acid |
| 5,20 Tocopherol |

### Herstellung:

Alle Zutaten mischen. Die Mischung klar schmelzen bei 85°C. Sofort in die Form ausgiessen.

### Anwendungsbeispiele: Peeling-Cremes, Typ O/W

| Phase A | |
|---|---|
| 3,00 | Ceteareth-6 |
| 1,50 | Ceteareth-25 |
| 3,00 | Glyceryl Stearate |
| 5,00 | Cetearyl Alcohol, Sodium Cetearyl Sulfate |
| 6,00 | Cetearyl Octanoate |
| 6,00 | Mineral Oil |
| 0,20 | Bisabolol |

| Phase B | |
|---|---|
| 2,00 | Propylene Glycol |
| 0,10 | Disodium EDTA |
| 3,00 | Polymerisat gemäß Herstellbeispiel 1 bis 7 |
| q.s. | Konservierungsmittel |
| 59,70 | dest. Wasser |

| Phase C | |
|---|---|
| 0,50 | Tocopheryl Acetate |
| q.s. | Parfümöl |

| Phase D | |
|---|---|
| 10,00 | Polyethylene |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Anschließend Phase D unterrühren.

### Anwendungsbeispiele: Rasierschäume

| | |
|---|---|
| 6,00 | Ceteareth-25 |
| 5,00 | Poloxamer 407 |
| 52,00 | dest. Wasser |
| 1,00 | Triethanolamine |
| 5,00 | Propylene Glycol |
| 1,00 | PEG-75 Lanolin Oil |
| 5,00 | Polymerisat gemäß Herstellbeispiel 1 bis 7 |
| q.s. | Konservierungsmittel |
| q.s. | Parfümöl |
| 25,00 | Sodium Laureth Sulfate |

### Herstellung:

Alles zusammenwiegen, danach rühren bis gelöst. Abfüllung: 90 Teile Wirksubstanz und 10 Teile Propan/Butan-Mischung 25:75.

### Anwendungsbeispiele: After Shave Balsam

| Phase A | |
|---|---|
| 0,25 | Acrylates/C10-30 Alkyl Acrylate Crosspolymer |
| 1,50 | Tocopheryl Acetate |
| 0,20 | Bisabolol |
| 10,00 | Caprylic/Capric Triglyceride |
| q.s. | Parfümöl |
| 1,00 | PEG-40 Hydrogenated Castor Oil |

| Phase B | |
|---|---|
| 1,00 | Panthenol |
| 15,00 | Alcohol |
| 5,00 | Glycerin |
| 0,05 | Hydroxyethyl Cellulose |
| 1,92 | Polymerisat gemäß Herstellbeispiel 1 bis 7 |
| 64,00 | dest. Wasser |

| Phase C | |
|---|---|
| 0,08 | Sodium Hydroxide |

### Herstellung:

Die Komponenten der Phase A mischen. Phase B unter Homogenisieren in Phase A einrühren, kurz nachhomogenisieren. Mit Phase C neutralisieren und erneut homogenisieren.

### Anwendungsbeispiele: Körperpflegecremes

| Phase A | |
|---|---|
| 2,00 | Ceteareth-6 |
| 2,00 | Ceteareth-25 |
| 2,00 | Cetearyl Alcohol |
| 3,00 | Glyceryl Stearate SE |
| 5,00 | Mineral Oil |
| 4,00 | Jojoba (Buxus Chinensis) Oil |
| 3,00 | Cetearyl Octanoate |
| 1,00 | Dimethicone |
| 3,00 | Mineral Oil, Lanolin Alcohol |

| Phase B | |
|---|---|
| 5,00 | Propylene Glycol |
| 0,50 | Veegum |
| 1,00 | Panthenol |
| 1,70 | Polymerisat gemäß Herstellbeispiel 1 bis 7 |
| 6,00 | Polyquaternium-44 |
| q.s. | Konservierungsmittel |
| 60,80 | dest. Wasser |

| Phase C | |
|---|---|
| q.s. | Parfümöl |

### Herstellung:

Die Phasen A und B getrennt auf ca. 80°C erwärmen. Phase B homogenisieren.
Phase B unter Homogenisieren in Phase A einrühren, kurz nachhomogenisieren.
Abkühlen auf ca. 40°C, Phase C hinzugeben und nochmals kurz homogenisieren.

### Anwendungsbeispiele: Zahnpasten

| Phase A | |
|---|---|
| 34,79 | dest. Wasser |
| 3,00 | Polymerisat gemäß Herstellbeispiel 1 bis 7 |
| 0,30 | Konservierungsmittel |
| 20,00 | Glycerin |
| 0,76 | Sodium Monofluorophosphate |

| Phase B | |
|---|---|
| 1,20 | Sodium Carboxymethylcellulose |

| Phase C | |
|---|---|
| 0,80 | Aromaöl |
| 0,06 | Saccharin |
| 0,10 | Konservierungsmittel |
| 0,05 | Bisabolol |
| 1,00 | Panthenol |
| 0,50 | Tocopheryl Acetate |
| 2,80 | Silica |
| 1,00 | Sodium Lauryl Sulfate |
| 7,90 | Dicalciumphosphate Anhydrate |
| 25,29 | Dicalciumphosphate Dihydrate |
| 0,45 | Titanium Dioxide |

### Herstellung:

Phase A lösen. Phase B in Phase A einstreuen und lösen. Phase C zugeben und unter Vakuum bei RT ca. 45 Min. rühren lassen.

### Anwendungsbeispiele: Mundwasser

| Phase A | |
|---|---|
| 2,00 | Aromaöl |
| 4,00 | PEG-40 Hydrogenated Castor Oil |
| 1,00 | Bisabolol |
| 30,00 | Alcohol |

| Phase B | |
|---|---|
| 0,20 | Saccharin |
| 5,00 | Glycerin |
| q.s. | Konservierungsmittel |
| 5,00 | Poloxamer 407 |
| 0,5 | Polymerisat gemäß Herstellbeispiel 1 bis 7 |
| 52,30 | dest. Wasser |

### Herstellung:

Phase A und Phase B getrennt klar lösen..Phase B in Phase A einrühren.

### Anwendungsbeispiele: Prothesenhaftmittel

| Phase A | |
|---|---|
| 0,20 | Bisabolol |
| 1,00 | Beta-Carotene |
| q.s. | Aromaöl |
| 20,00 | Cetearyl Octanoate |
| 5,00 | Silica |
| 33,80 | Mineral Oil |

| Phase B | |
|---|---|
| 5,00 | Polymerisat gemäß Herstellbeispiel 1 bis 7 |
| 35,00 | PVP (20%ige Lösung in Wasser) |

### Herstellung;

Phase A gut mischen. Phase B in Phase A einrühren. Anwendungsbeispiel 32: Hautpflegecreme, Typ O/W

| Phase A | |
|---|---|
| 8,00 | Cetearyl Alcohol |
| 2,00 | Ceteareth-6 |
| 2,00 | Ceteareth-25 |
| 10,00 | Mineral Oil |
| 5,00 | Cetearyl Octanoate |
| 5,00 | Dimethicone |

| Phase B | |
|---|---|
| 3,00 | Polymerisat gemäß Herstellbeispiel 1 bis 7 |
| 2,00 | Panthenol, Propylene Glycol |
| q.s. | Konservierungsmittel |
| 63,00 | dest. Wasser |

| Phase C | |
|---|---|
| q.s. | Parfümöl |

### Herstellung:

Phase A und B getrennt auf ca. 80 C erwärmen. Phase B in Phase A unter Homogenisieren einrühren, kurz nachhomogenisieren. Abkühlen auf ca. 40 C, Phase C hinzugeben, nochmals homogenisieren.

### Anwendungsbeispiele: Hautpflegecremes, Typ W/O

| Phase A | |
|---|---|
| 6,00 | PEG-7 Hydrogenated Castor Oil |
| 8,00 | Cetearyl Octanoate |
| 5,00 | Isopropyl Myristate |
| 15,00 | Mineral Oil |
| 2,00 | PEG-45/Dodecyl Glycol Copolymer |
| 0,50 | Magnesium Stearate |
| 0,50 | Aluminum Stearate |

| Phase B | |
|---|---|
| 3,00 | Glycerin |
| 3,30 | Polymerisat gemäß Herstellbeispiel 1 bis 7 |
| 0,70 | Magnesium Sulfate |
| 2,00 | Panthenol |
| q.s. | Konservierungsmittel |
| 48,00 | dest. Wasser |

| Phase C | |
|---|---|
| 1,00 | Tocopherol |
| 5,00 | Tocopheryl Acetate |
| q.s. | Parfümöl |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren.

### Anwendungsbeispiele: Lippenpflegecremes

| Phase A | |
|---|---|
| 10,00 | Cetearyl Octanoate |
| 5,00 | Polybutene |

| Phase B | |
|---|---|
| 0,10 | Carbomer |

| Phase C | |
|---|---|
| 2,00 | Ceteareth-6 |
| 2,00 | Ceteareth-25 |
| 2,00 | Glyceryl Stearate |
| 2,00 | Cetyl Alcohol |
| 1,00 | Dimethicone |
| 1,00 | Benzophenone-3 |
| 0,20 | Bisabolol |
| 6,00 | Mineral Oil |

| Phase D | |
|---|---|
| 8,00 | Polymerisat gemäß Herstellbeispiel 1 bis 7 |
| 3,00. | Panthenol |
| 3,00 | Propylene Glycol |
| q.s. | Konservierungsmittel |
| 54,00 | dest. Wasser |

| Phase E | |
|---|---|
| 0,10 | Triethanolamine |

| Phase F | |
|---|---|
| 0,50 | Tocopheryl Acetate |
| 0,10 | Tocopherol |
| q.s. | Parfümöl |

### Herstellung:

Phase A klar lösen. Phase B dazugeben und homogenisieren. Phase C addieren und schmelzen bei 80 C. Phase D auf 80 C erwärmen. Phase D zu Phase ABC geben und homogenisieren. Abkühlen auf ca. 40 C, Phase E und Phase F zugeben, nochmals homogenisieren.

### Anwendungsbeispiele: Glänzende Lippenstifte

| Phase A | |
|---|---|
| 5,30 | Candelilla (Euphorbia Cerifera) Wax |
| 1,10 | Bees Wax |
| 1,10 | Microcrystalline Wax |
| 2,00 | Cetyl Palmitate |
| 3,30 | Mineral Oil |
| 2,40 | Castor Oil, Glyceryl Ricinoleate, Octyldodecanol, Carnauba, |
| | Candelilla Wax, |
| 0,40 | Bisabolol |
| 16,00 | Cetearyl Octanoate |
| 2,00 | Hydrogenated Coco-Glycerides |
| q.s. | Konservierungsmittel |
| 1,00 | Polymerisat gemäß Herstellbeispiel 1 bis 7 |
| 60,10 | Castor (Ricinus Communis) Oil |
| 0,50 | Tocopheryl Acetate |

| Phase B | |
|---|---|
| 0,80 | C. I. 14 720:1, Acid Red 14 Aluminum Lake |

| Phase C | |
|---|---|
| 4,00 | Mica, Titanium Dioxide |

### Herstellung:

Die Komponenten der Phase A einwiegen und aufschmelzen. Phase B homogen einarbeiten. Phase C zugeben und unterrühren. Unter Rühren auf Raumtemperatur abkühlen.

## Patentansprüche

1. Pfropfcopolymerisate, die erhältlich sind durch radikalische Pfropfcopolymerisation von
a) N-Vinylformamid,
b) eines oder mehreren copolymerisierbaren Monomeren der allgemeinen Formel (I) 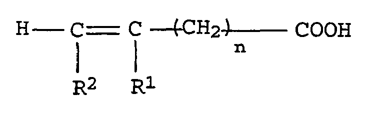
wobei n= 0,1 und R¹ und R² unabhängig voneinander = H, C₁-C₄ Alkyl, CN, COOH darstellen,
und/oder einer oder mehreren ungesättigten Sulfonsäuren
und/oder einer oder mehreren ungesättigten Phosphonsäuren
auf eine polymere Pfropfgrundlage c) der allgemeinen Formel

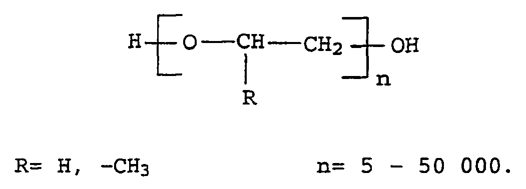

2. Pfropfpolymerisate nach Anspruch 1, **dadurch gekennzeichnet, daß** die radikalische Pfropfcopolymerisation in Gegenwart eines Vernetzers d) durchgeführt wird.

3. Pfropfpolymerisate nach Anspruch 1, **dadurch gekennzeichnet, daß** man als Monomer b) Verbindungen der Formel (I) einsetzt.

4. Pfropfpolymerisate nach Anspruch 1 und/oder 2, **dadurch gekennzeichnet, daß** man eine polymere Pfropfgrundlage c) mit einem Molekulargewicht von 500 bis 50 000, insbesondere von 5000 bis 40 000 einsetzt.

5. Verwendung von Pfropfpolymerisaten nach mindestens einem der Ansprüche 1 bis 4 in kosmetischen Mitteln, insbesondere in haarkosmetischen Mitteln.

6. Verwendung nach Anspruch 5 als Verdicker.

## Claims

1. A graft copolymer which is obtainable by free radical graft copolymerization of
a) N-vinylformamide,
b) one or more copolymerizable monomers of the formula (I) 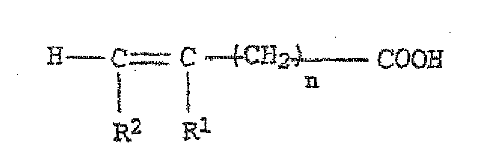
where n is 0 or 1 and R¹ and R², independently of one another, are H, C₁-C₄-alkyl, CN or COOH,
and/or one or more unsaturated sulfonic acids
and/or one or more unsaturated phosphonic acids
onto a polymeric grafting base c) of the formula

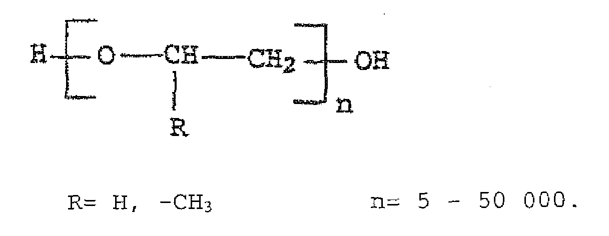

2. The graft polymer according to claim 1, wherein the free radical graft copolymerization is carried out in the presence of a crosslinking agent d).

3. The graft polymer according to claim 1, wherein the monomer b) used is a compound of the formula (I).

4. The graft polymer according to claim 1 and/or 2, wherein a polymeric grafting base c) having a molecular weight of from 500 to 50 000, in particular from 5000 to 40 000, is used.

5. The use of graft polymers according to at least one of claims 1 to 4 in cosmetic compositions, in particular in hair cosmetics.

6. The use according to claim 5 as a thickener.

## Revendications

1. Copolymères greffés qu'on obtient par copolymérisation par greffe radicalaire de :
a) N-vinylformamide
b) un ou plusieurs monomères copolymérisables de formule générale (I) 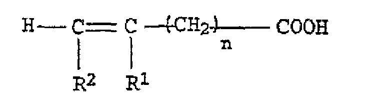
dans laquelle n = 0, 1 et R¹ et R² représentent indépendamment l'un de l'autre H, alkyle en C₁-C₄, CN, COOH,
et/ou un ou plusieurs acides sulfoniques insaturés
et/ou un ou plusieurs acides phosphoniques insaturés
sur une base de greffe polymère c) de formule générale 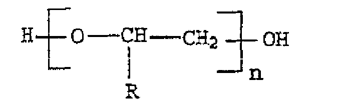 R= H, -CH₃
n= 5 à 50 000

2. Polymères greffés selon la revendication 1, **caractérisés en ce que** la copolymérisation par greffe radicalaire a lieu en présence d'un agent de réticulation d).

3. Polymères greffés selon la revendication 1, **caractérisés en ce qu'**on utilise comme monomère b) des composés de formule (I).

4. Polymères greffés selon la revendication 1 et/ou 2, **caractérisés en ce qu'**on utilise une base de greffe polymère c) d'un poids moléculaire allant de 500 à 50 000, en particulier de 5000 à 40 000.

5. Utilisation de polymères greffés selon au moins une des revendications 1 à 4 dans des produits cosmétiques, en particulier dans des produits cosmétiques pour cheveux.

6. Utilisation selon la revendication 5 comme épaississant.
